# EUROPEAN PATENT APPLICATION

(11) **EP 3 031 486 A1**
(43) Date of publication of application: **15.06.2016**
(21) Application number: 14197033.5
(22) Date of filing: 09.12.2014
(51) Int. Cl.: A61M 5/50, A61J 1/16, A61M 5/24

(54) **Medication cartridges for optimised dosing**

(71) Applicant: CONARIS Research Institute AG, 24118 Kiel (DE)
(72) Inventor: Wätzig, Georg, 24106 Kiel (DE); Seegert, Dirk, 24229 Dänischenhagen (DE)
(74) Representative: Eisenführ Speiser

(57) **Abstract**

The present invention relates to a cartridge that may be a unit for an administration device for medication, such as, *e.g.,* an injector, a pen, an inhalator or a dispenser. The cartridge of the invention enables personalized dosing, and becomes inactivated in case of breaches of cartridge integrity, unauthorised use and/or after use. It preferably enables the extension of the cold chain surveillance to the actual site and time of administration, tracking and tracing. The present invention further enables the interaction of the cartridge with a suitable administration device, leading to completely safe, authorised, traceable, variable, individualised, programmable, remote controlled and/or two-way documentable dosing of administered drug formulations with the added benefit of allowing remote controlled, tamper-proof flat pricing models for variable dosing per patient and time unit.

## Description

### FIELD OF THE INVENTION

The present invention relates to a cartridge that may be a unit for an administration device for medication, such as, e.g., an injector, a pen, an inhalator or a dispenser. The cartridge of the invention enables personalized dosing, and becomes inactivated in case of breaches of cartridge integrity, unauthorised use and/or after use. It preferably enables the extension of the cold chain surveillance to the actual site and time of administration, tracking and tracing. The present invention further enables the interaction of the cartridge with a suitable administration device, leading to completely safe, authorised, traceable, variable, individualised, programmable, remote controlled and/or two-way documentable dosing of administered drug formulations with the added benefit of allowing remote controlled, tamper-proof flat pricing models for variable dosing per patient and time unit.

### BACKGROUND

Personalized dosing, in particular by self-administration, of medications by using, e.g., injection or inhalation devices is becoming increasingly important and will be at the heart of the personalized medicine of the 21^{st} century. In this regard, physicians and patients will have to adapt doses according to the state of disease and/or pharmacokinetic and/or pharmacodynamic parameters, which may require individual doses that are different at each time of administration and that are dependent on complex algorithms to calculate adjustments using biomarkers, drug levels and/or other parameters. The "internet of things" is on the verge of revolutionising medicine not only by enabling seamless tracking and inventory of single medication units along the complete supply and storage chain, but also as "connected health" by connecting patient-operated monitoring and application devices to, e.g., smartphones or other personal assistance devices as well as databases and healthcare specialists for two-way tracking and evaluation. In addition to the already well-established smartphone-enabled health and fitness devices, "mobile health" apps and electronic product (training) support, the connected health approach encompasses multidirectional internet and/or mobile communication between medical and laboratory specialists, caregivers and patients without the immediate need for face-to-face interaction.

The vanguard in terms of technology and user optimisation were and still are the diverse injector solutions for administering insulin to patients with diabetes. The main driver for the innovative force in this sector is the challenge of administering personalized doses (insulin) based on user measurements of a direct target for drug efficacy (blood glucose levels) several times a day without the possibility of permanent guidance and supervision by an attending physician. For insulin therapy, it becomes important to measure the target several times daily in a continuous way and to record the efficacy and quantity of each insulin injection in order to assess the long-term benefits of therapy.

Re-usable pens and other drug delivery devices have been equipped for some years with embedded electronics, e.g., for dose settings and tracking of injection dates (Bauss 2014: Connecting Drug Delivery Devices to Smartphone Applications and Mobile Medical Apps; PDA Universe of Pre-filled Syringes and Injection Devices; Huntington Beach CA; October 2014). One example for such a pen is the HumaPen^{®} Memoir (Lilly), which saves the dose, date and time of the last 16 insulin injections in order to avoid double injections and to allow both the patient and the attending physician to look up the last doses in the device, albeit without remote access. Smart injection devices like, e.g., KiCoPen (Cambridge Consultants), Bee+ (Vigilant) or the Pendiq^{®} insulin pen (Pendiq) support computer connections and programming or wirelessly report the injected amount of insulin back to a smartphone app. As the power supply for such devices can be a key differentiating factor, the KiCoPen has been designed to harvest energy from removal and application of the pen's cap, thus needing no battery or accumulator charging.

Examples for the prior art in the patent literature on automated drug delivery include insulin infusion devices featuring remote-sensing (blood glucose levels), manual input (meal intake) and user authentication (biometric analysis) (e.g., US 20120249294 A1), systems for integrating diverse parameters into patient care (e.g., US 20020169636 A1), apparatuses for titrating drug delivery (US 7,229,430 B2) and computer-controlled intravenous drug delivery systems (EP 1547631 A1). WO 2013115843 A1 describes a programmable apparatus for autonomous, variable rate, temperature-dependent delivery of a substance. In WO 2010096061 A1, intelligent sleeve containers for controlled syringe systems are presented. Marketed examples comprising some of the more patient-centered aspects of the patent literature are the MyGlucoHealth system (Entra Health Systems) or the iPhone-enabled glucose meter iBGStar (AgaMatrix/Sanofi-Aventis).

The BETACONNECT™ autoinjector (Bayer) enables patients with multiple sclerosis to customise their self-administration of Betaferon^{®} in terms of injection speed, needle insertion depth and injection reminders. Moreover, BETACONNECT™ also allows bluetooth-or cable-based upload of injection data to an app and a secure website to facilitate personalized support by healthcare professionals.

In the field of emergency epinephrin injectors used for anaphylaxis patients, the design study EpiShell by Hong Ying Guo for a novel epinephrine injector guidance system incorporates voice guidance to ensure proper injection, which is activated from a wirelessly connected smartphone once the EpiShell is opened. The corresponding app also offers emergency contact and refill or expiry date reminders.

Another facet of smart injection systems is exemplified by the Dynamic Pressure Sensing^{®} technology (Milestone Medical), which offers computer-controlled real-time feedback to the medical practitioner performing a hypodermic injection in order to optimise injection speed and needle insertion depth as well as to minimise injection-induced pain.

In the field of contrast media for radiology, MEDRAD's Certegra™ platform offers multiple possibilities for personalized injection protocols.

For subcutaneous (s.c.) administration of biological drugs (biologics) like monoclonal antibodies, single-use injectors are the current state of the art [*e*.*g*., the HUMIRA^{®} pen for administering the monoclonal anti-tumour necrosis factor antibody adalimumab (HUMIRA^{®}; AbbVie) or the wearable bolus injector SmartDose^{®} (West Pharmaceutical Services)]. The single-use syringe or injector solutions in the field of biologics are fixed regarding the administration dose in contrast to the self-administration pens for insulin. One of the reasons for the differing state of the art is the fact that the dosing decisions for biologics are usually made by physicians based on clinical symptoms and laboratory parameters. Usually, it is only after a visit in a hospital, an outpatient clinic or a private practice and after personal examination of the patient that a physician decides to prescribe or administer, e.g., one single-use injector or also two injectors, if heavy symptoms or low drug levels require to double the dose. Therefore, dynamic dose adaptation for biologics is not presently in use, and most injection systems deliver only a unified dose.

Thus, insulin is the prototype for a fast-acting (polypeptide) drug with a short half-life, whereas biologics are prototypes of long-acting drugs with long half-lives.

However, in this state of the art, several unsolved technical problems as well as unmet medical and economical needs remain.

Whereas patients with diabetes are used to permanently adapt their self-administered insulin doses to their blood glucose levels, patients with other chronic diseases - e.g., patients with chronic inflammatory diseases like rheumatoid arthritis or inflammatory bowel diseases - and their physicians are facing the unsolved problem of treating variable disease activities in patients with variable body weight and other parameters relevant for treatment using single-use injectors delivering only one particular dose. For example, plasma levels and efficacy of biologics in patients with inflammatory bowel diseases may vary strongly depending on the drug loss via the inflamed intestinal mucosa [see, *e*.*g*., section 2.3.3. (p. 13 f.) of the assessment report EMA/640422/2013 (25 July 2013) of the European Medicines Agency (EMA) on golimumab (Simponi^{®})]. In a severe phase of the disease, the attending physician may administer - or instruct the patient to use - two single-use injectors to double the dose, but a real-time adaptation to, *e*.*g*., biomarker or drug trough levels in the blood is not possible with the current state of art, which offers only a very rough approximation of context-dependent dosing. In contrast to insulin, the extremely high costs of biologics do not favour individual dose increases under the present administration techniques, as this would significantly increase the annual therapeutic costs in a chronic disease and change the assumptions of the pharmacoeconomic models used in price negotiations.

Currently, the variation of dosing costs for biologics and other medications due to the application of, *e*.*g*., no, one or two single-use injectors per patient every two weeks, depending on the disease status, is a major problem for budgeting and a major cost and uncertainty factor for third party payers in the healthcare sector. The pharmacoeconomic models supporting price negotiations compare the gains through improved quality of life leading to restored work productivity and decreased use of health care resources (*e*.*g*., hospitalisation days, operations, etc.) against the manufacturer's selling price. These are typically very high, with most health care systems seeing now extreme expenses for single biologics (Schreiber et al. 2014, Dtsch. Med. Wochenschr. 139:2399). The use of higher, individual doses violates the assumptions for therapy costs in such models. On the other hand, the introduction of a "flat rate" is technically very difficult, as it would require traceability of the administered dose through all economic partners of the health care system in order to guarantee a fair compensation for the manufacturer.

In general, self-administration of drugs performed weekly or less frequently is an important factor for patient adherence and compliance in a chronic disease, as the previous standard of care using mandatory intravenous infusions in hospitals, outpatient clinics or private practices much more severely affected quality of life. In addition, in cases where the administration of the drug is performed by the attending physician, usually two visits of the patient are required: one for drawing blood to determine all parameters necessary for a dosing decision, and a second visit for administering the actual dose, which increases healthcare costs and reduces the quality of life of the patient. In general, incompliance is a large concern in chronic diseases, *e*.*g*., in inflammatory bowel diseases (Kane 2008, Drugs 68:2601; Kane et al. 2009, Adv. Ther. 26:936; Kane & Robinson 2010, Aliment. Pharmacol. Ther. 32:1051). In some studies, less than 40% of patients take their chronic medication needed to stabilize the disease. In these studies, it is assumed that most of the relapses may result from incomplete or incorrect self-administration of drugs.Therefore, there is a large unmet need for flexible dosing, preferably by self-administration, based on multiple decision parameters, such as current symptoms, biomarker levels and/or drug levels, but still under the control of the attending physician.

In addition, as counterfeit medications are on the rise worldwide, current legislation both in the EU and in the US is focusing on trackability and traceability of pharmaceuticals along the complete value and distribution chain. The use of one or more identification (ID) elements like barcodes, two-dimensional (2D) barcodes, 2D data matrix codes, and/or radio-frequency identification (RFID) tags for such so-called ePedigrees of product units offer numerous possibilities and layers of security to provide tamper-proof and unique identification of each unit of medication (*e*.*g*., of cartridges of the present invention) as well as feedback to the manufacturer via the internet when the unit is used in a suitable device connected to the internet. The corresponding technologies are well known in the art and also used in other contexts, *e*.*g*., in automatic ID control of ink cartridges for printers or manual ID verification by camera-based code-reading smartphone apps or multimedia messaging services which may transmit photographs of the ID code taken by the end user to the manufacturer and/or other links of the supply chain. However, the clinical reality even in developed countries is that printed adhesive labels are manually scanned to document the administration of a certain medication unit to a certain patient, which is an error-prone and laborious way of pharma tracking and tracing and does not directly support real-time tracking and tracing, but only serves for paper documentation. In addition, tracking through wholesalers and pharmacies is often difficult as these are allowed to buy from various sources including the import from other countries in some jurisdictions (*e*.*g*., in the European Union). It is difficult to connect single prescriptions with the actual purchase trail of the medication delivered to fill the prescription. In case of very expensive or easily damageable medications, this is an important issue and an unsolved problem.

Surveillance of the cold chain currently ends when the medication cartridge leaves the pharmacy or medical facility (*e*.*g*., the hospital, outpatient clinic or private practice) after transfer to the end user (patient) or an authorised person. The authorities plan to extend cold chain surveillance (see, *e*.*g*., the European Commission Guideline of 5 November 2013 on Good Distribution Practice of medicinal products for human use, 2013/C 343/01), but many technical problems associated with this desired extension remain unsolved.

State-of-the-art technologies like RFID sensor tags, miniature temperature logging devices and/or chemical reactions creating colour changes to indicate whether the cartridge's temperature has left a predefined window could theoretically enable the end user (patient) to determine by visual inspection and/or by interaction with suitable devices whether or not the cartridge has been stored under acceptable conditions from the moment of release by the manufacturer until the moment of quality control prior to administration. For many biologics, this is a decisive quality and safety feature, but there is currently no suitable technical solution.

Depending on the context, RFID can use active, passive or semi-passive tags, close coupling tags for direct interaction, remote coupling tags for intermediate-range interaction or long-range RFID systems for logistics. In many cases, a combination of barcode and RFID technologies are used for so-called smart labels or electronic shelf labels.

Today, RFID tagging at the primary packaging level is practically non-existent in the pharmaceutical industry (Kania 2012: R.I.P. for RFID? Healthcare Packaging, Nov 8. 2012). The reasons for this are not only cost issues, but also technical challenges. For example, physically finding (not only detecting the presence of) single units with conventional readers in an environment where many such units are stored can be difficult due to the multitude of response signals, even though the rapid overview of all stored items using RFID is superior to other technologies (Kania 2012: R.I.P. for RFID? Healthcare Packaging, Nov 8. 2012). However, to meet ePedigree requirements as defined by the legislators, *e*.*g*., by the California Board of Pharmacy Track and Trace Initiative, every single unit in every packaging unit would have to be trackable and traceable, which can be next to impossible without RFID or similar no-contact technologies. For example, if every single dose item had to be taken out of its packaging unit to be inventoried by, *e*.*g*., scanning barcodes or data matrix codes, other packaging seals and safety measures could be violated, it could take an enormous amount of time and - last, but not least - such physical interaction may cause errors and damage. In addition, temperature monitoring is one of the key areas in which RFID technology is hard to beat in a clinical or pharmaceutical setting (Kania 2012: R.I.P. for RFID? Healthcare Packaging, Nov 8. 2012), and the miniaturisation of the corresponding RFID sensor systems allows sensor tagging on the single item level.

In 2004, the US Food and Drug Administration (FDA) issued guidelines for the use of RFID, which excluded biologics due to insufficient data on potentially harmful effects of RFID systems on biologics (Compliance Policy Guide Section 400.210). In the same document, the FDA defined rules for studies to demonstrate the non-interference of RFID with particular products, which would also have to be followed in the context of a development project. The corresponding pilot programme is scheduled to end on 31 December 2014 (Federal Register Vol. 77, No. 242, pp. 74668-74669; 17 Dec. 2012). Meanwhile, many studies and a large collaborative research effort of academic and industrial partners have demonstrated that RFID has no non-thermal effects on biologics and that the heating effect is negligible (Uysal et al. 2012, PDA J. Pharm. Sci. Technol. 66:333). To the Applicant's knowledge, the FDA has not yet officially changed its view, whereas other authorities like the EMA have not issued any guidelines in this matter, probably due to the insignificant risk and the rather clear data situation. Nevertheless, the regulatory and practical requirements outlined above will be hard to meet without RFID single-unit tagging, and it is expected that also the FDA will accept RFID tagging under defined conditions after reviewing all data gathered in the pilot programme period.

In summary, the problem of safe and personalized dosing with several layers of control both locally and remotely at the level of single administration units like medication cartridges has, to the Applicant's knowledge, not been solved before. Moreover, the teachings and development trends of the prior art point away from the invention described herein. Accordingly, the technical problem underlying the present invention is to provide novel medication cartridges for optimised dosing by administration devices, in particular by self-administration devices.

The solution to said technical problem is achieved by providing the embodiments characterized in the claims.

### SUMMARY OF THE INVENTION

The object of the present invention is to provide novel medication cartridges that may comprise an administration unit or that may be a unit for an administration device, e.g., an injector and/or a pen and/or an inhalator and/or a dispenser. It is envisioned that the cartridge of the present invention may be used with different types and models administration devices, as long as the specifications for the interaction between the cartridge and the device are observed. The administration can be advantageously performed by a patient (self-administration) or by healthcare specialists like, e.g., the attending physician. It is a further object of the invention to improve drug safety.

According to the invention, the above problem is solved by the claimed medication cartridge which
(a) contains sufficient drug formulation for the execution of an administration programme devised for the drug and the medical condition to be treated;
(b) comprises means to enable programmed administration of a personalized dose of the drug formulation to a patient, comprising the use of the cartridge in one or more administration events;
(c) comprises means to (preferably irreversibly) invalidate and/or inactivate the cartridge (i) after use according to (b) and/or (ii) by any breach of cartridge integrity and/or (iii) by unauthorised use, or comprises means to activate the cartridge if none of (i) - (iii) is detected;
(d) is adapted for use together with a suitable administration device or comprises an administration unit.

Preferably the cartridge according to the invention comprises at least one unique identifier such as, *e*.*g*., a code (consisting of any element from the group of numbers, letters and/or symbols), a barcode, a 2D barcode, a 2D data matrix code, an RFID tag and/or NFC technology, which enable tracking and tracing of the cartridges from the moment of release by the manufacturer of the cartridge until the moment of quality control prior to administration;

In addition, the cartridge of according to the invention preferably comprises (*e*.*g*., if required by the storage specifications of the drug formulation contained in the cartridge), means for extending of the temperature (*e*.*g*., cold chain) surveillance beyond the storage at a pharmacy or hospital or outpatient clinic or medical practice to the actual site and time of administration like, *e*.*g*., RFID sensor tags, temperature logging devices and/or chemical reactions creating visible colour and/or turbidity changes to indicate whether a cartridge's temperature has left a predefined window;

Additionally preferred is a cartridge according to the invention comprising means for indication of
1. a breach of the integrity of the cartridge and/or its content (*e*.*g*., by physical force, manipulation or tampering) and/or
2. if applicable, a breach of a predefined temperature window (*e*.*g*., a breach of the cold chain) and/or
3. prior use of a cartridge in another administration device and/or in the same administration device beyond a predefined time window and/or
4. unauthorised use,
by preferably irreversible mechanical and/or physical and/or chemical and/or optical and/or electrical and/or electronic and/or digital and/or data indicators and/or mechanisms, such as, *e*.*g*., a disturbance of the mechanical and/or physical and/or electrical and/or electronic interaction between the cartridge and the administration device or administration unit, a colour reaction or turbidity in viewing window(s), a (preferably irreversible) indicator label, a (preferably irreversible) thermochromic indicator, and/or information in a data unit such as an RFID (sensor) tag;

Preferably the cartridge of the present invention is adapted for interaction with suitable administration devices or administration units enabling
1. administration of a drug formulation contained in the cartridge to a particular patient, wherein the dose is dependent of one or more unique patient identifiers (*e*.*g*., biometric identifiers such as a fingerprint scan performed, *e*.*g*., by the administration device), and/or
2. administration of a personalized dose of the drug formulation contained in the particular cartridge to a particular patient based on a disease symptom and/or a laboratory parameter, *e*.*g*., drug level and/or a biomarker e.g. from the patient's blood, urine, stool, breath or tissue that have been previously measured by any device (including, but not limited to, point of care devices and/or devices used by the patient), and/or
3. administration of a personalized dose of the drug formulation contained in the particular cartridge to a particular patient, wherein the dose is defined and/or qualified and/or cleared and/or directly controlled and/or remote-controlled by the attending physician and/or other healthcare specialists and/or computer programmes and/or computer algorithms, and/or
4. variable, personalized dosing based on information transmitted by the attending physician and/or other healthcare specialists and/or computer programmes and/or computer algorithms to the administration device and wherein the cartridge contains sufficient drug formulation for the execution of a personalized administration programme devised for the patient, the drug and the medical condition to be treated, *e*.*g*., any dose up to the maximum dose defined by the appropriate guidelines and/or healthcare authorities and/or the attending physician and/or other healthcare specialists and/or computer programmes and/or computer algorithms, and/or
5. a single administration or two or more preferably immediately consecutive administrations per cartridge and wherein each administration event (*e*.*g*., several consecutive injections in order to distribute a volume too large for one injection site) is in a predefined time window and/or defined by the administration programme for optimal dosing, and/or
6. tracking and tracing of the cartridge from the time of release from the manufacturer of the cartridge until the time of administration, and/or
7. (Preferably irreversible) invalidation and/or inactivation of the cartridge if the cartridge integrity has been compromised and/or if the cartridge's temperature has left a predefined window and/or in case of prior use of the cartridge in another administration device and/or in case of prior use in the same administration device beyond a predefined time window and/or in case of unauthorised use, thereby preventing any potentially unsafe administration, and/or
8. transmission of information via the administration device directly or indirectly back to the attending physician and/or other healthcare specialists and/or a hospital and/or a medical office and/or the manufacturer of the cartridge and/or the manufacturer of the administration device and/or healthcare authorities and/or third party payers and/or medical or scientific or insurance databases in order to document the administration event and/or protection of the patient's identity and data by suitable coding and anonymisation technologies, and/or
9. protection of the patient's identity and data by suitable coding and anonymisation technologies.

In summary, the present invention enables and includes the use of the cartridges of the present invention together with suitable administration devices or administration units allowing, if needed, personalized, variable, safe, trackable, traceable, programmable and/or two-way documentable dosing of administered drug formulations under the direct or remote control and/or clearance of the attending physician and/or other healthcare specialists and/or computer programmes and/or computer algorithms with the added benefit of allowing tamper-proof flat pricing models for variable dosing per patient and time unit.

### DETAILED DESCRIPTION

Herein, the words "preferred" or "preferably" refer to embodiments that may have certain benefits under certain circumstances, but other embodiments may also be preferred under the same or other circumstances. The recitation of one or more preferred embodiments does not imply exclusion of other useful embodiments from the scope of the invention. Terms like "comprises" and variations thereof do not have a limiting meaning in the description and claims in the sense that no further feature may be comprised. Citation of certain sections of documents from the literature does not imply that the rest of such documents is not relevant or not incorporated by reference. The recitations of numerical ranges by one or two endpoints includes all numbers subsumed within that range (*e*.*g*., "1 to 10" includes 1, 2.4, 4.576, etc., and "lower than 1" includes all numbers smaller than 1). For any method disclosed or cited herein that includes discrete steps, the steps may be conducted in any feasible order, and any combination of two or more steps may be conducted simultaneously. Any example or list of examples should not be interpreted as a restriction of any kind or as an exclusive list.

The methodological elements for enabling the claimed invention are well known in the art. All techniques and methods mentioned and referred to in the present application are merely examples and shall not be construed as restrictions of any kind.

As used herein, the term "administration" pertains to any introduction into or application onto the body of a patient. The drug formulation contained in a cartridge of the present invention may be administered by various routes of administration, including, but not limited to, injection, inhalation, infusion or ingestion. The administration may be performed by the patient (self-administration) in the presence or absence of healthcare specialists (*e*.*g*., the attending physician) or by a healthcare specialist. For example, a patient may administer the drug formulation contained in the cartridge by using a suitable administration device, or a physician may use such an administration device in the same or another fashion, or a physician may use a dispensing device to produce an infusion solution personalised to the patient after unlocking of the patient's personal administration programme in the dispensing device by the patient using one or more unique personal identifiers according to the invention. Administration modes may also vary during treatment. For example, induction treatment of a patient may start with one or more intraveneous infusions administered by healthcare specialists after using an patient-specific administration programme for a dispensing device as described above, whereas maintenance treatment may be performed by the patient at home, *e*.*g*., by subcutaneous injection using a different administration programme in a self-injector according to the present invention.

As used herein, the term "administration device" pertains to any device or machinery which is used to execute a personalized administration programme for administering to a patient a personalized dose of a drug formulation contained in a cartridge according to the present invention. Such devices include, but are not limited to, injectors and/or pens and/or inhalators and/or dispensers. The personalization of the administration is advantegeously achieved by the mandatory use of a personalized administration programme.

As used herein, the term "administration unit" pertains to any unit that is part of the cartridge according to the invention and is able to perform any of the functions of an "administration device" as described above.

As used herein, the term "administration programme" pertains to any information (*e*.*g*., executable code, non-executable specifications, data in text format and the like) controlling an administration device or an administration unit and informing its user to administer a personalized dose to a particular patient from a cartridge of the present invention, preferably after passing one or more quality tests. Such an administration programme may be, *e*.*g*., downloaded from an authorised website or transmitted via mobile, wireless, wired or any other means of communication to the administration device prior to administration. The use of the administration programme is preferably personally enabled by the patient by entering one or more unique identifiers to verify the identity of the patient. The administration programme may define one or more administration events in predefined time windows and intervals. The administration programme may be modified, interrupted or invalidated by the administration device or by any remote-controlling entity, *e*.*g*., the attending physician or computer databases, if novel information not available at the time of first transmission of the administration programme indicates to change or stop the programme.

As used herein, the term "administration event" pertains to a single administration or two or more consecutive administrations using a cartridge according to the invention in a predefined time window. For example, an injection device according to the present invention may administer two consecutive injections (*e*.*g*., one into each thigh) of a biologic drug within a time window of, *e*.*g*., one minute. The time window of one administration may be any period from one second to 24 hours, preferably one second to 15 minutes. In exceptional situations, it is also possible that one administration event may not be sufficient for a specific dose requirement because the administration programme may require more than the drug formulation contained in one cartridge. Here, it may be possible that more than one cartridge may be used in a "combined administration event", wherein the summed-up dose from the two or more cartridges involved in the combined administration event is controlled, *e*.*g*., by the administration device, a remote controller in contact with the cartridge(s) and/or by programming of the use of each cartridge involved, *e*.*g*., by the attending physician.

As used herein, the term "cartridge integrity" pertains to all aspects of the cartridge which ensure that the cartridge itself and the drug formulation contained therein are delivered from the manufacturer of the cartridge to the end user (patient) without any loss of quality beyond predefined specification windows, including, but not limited to, identity, validity, correct identification with full trackability and traceability, exclusion of tampering, correct storage conditions along the whole supply chain, exclusion of physical or chemical changes of the cartridge and the drug formulation contained therein and exclusion of unintended or unauthorised changes to data stored in or on the cartridge.

As used herein, the term "unauthorised use" pertains to any use of a cartridge of the present invention beyond the scope of use defined by the manufacturer and/or the healthcare specialists performing or supervising the administration, *e*.*g*., use in an unauthorised injection device (*e*.*g*., in a counterfeit device or in a device trying to override safety and/or ownership and/or payment and/or reporting technologies incorporated into the cartridge).

Part of the present invention are novel medication cartridges for administration devices, e.g., injectors and/or pens and/or inhalators and/or dispensers. In the context of self-administration, the cartridges of the present invention are vital for controlling that a particular patient can self-administer a particular and quality-controlled dose of a drug formulation contained in the cartridge. Preferably, the technologies described herein assist healthcare specialists in administering a correct and safe personalized dose to a patient. Application of this particular dose is performed by suitable administration devices or administration units, which may function as a communication and safety hub between the patient and one or more of the following parties: the attending physician, other healthcare specialists, a hospital, a medical office, the manufacturers of the cartridge and/or of the administration device, healthcare authorities, third party payers and/or medical or scientific or insurance databases. In particular, the cartridges of the present invention can ensure that only an authorised administration programme can be executed in an authorised administration device to administer one or more personalized doses to one particular patient. Depending on the adminstration programme, the administration may be performed in one or more administration events per cartridge or may require the use of one or more cartridges in one or more administration events.

Thus, the present invention provides for a medication cartridge which
(a) contains sufficient drug formulation for the execution of an administration programme devised for the drug and the medical condition to be treated;
(b) comprises means to enable programmed administration of a personalized dose of the drug formulation to a patient, comprising the use of the cartridge in one or more administration events;
(c) comprises means to (preferably irreversibly) invalidate and/or inactivate the cartridge (i) after use according to (b) and/or (ii) by any breach of cartridge integrity and/or (iii) by unauthorised use, or comprises means to activate the cartridge if none of (i) - (iii) is detected;
(d) is adapted for use together with a suitable administration device or comprises an administration unit.

Tamper-evident surveillance technologies are an important preferred aspect of the present invention. This does not only apply to the identification information of the cartridge or an optional tamper-proof temperature logging system (see below), but also to the contents of the cartridge. If a breach of the cartridge's integrity occurs, regardless of the exact construction of the particular embodiment of the invention and regardless of the cause or intention behind the tampering (*e*.*g*., accidental damage, physical force, manipulation, exchange or removal of the cartridge's contents, breach of the defined temperature thresholds, use in an unauthorised administration device or breach of other predefined safety or quality parameter limits), this breach of integrity may be indicated by, *e*.*g*., one or more preferably irreversible mechanical and/or physical and/or chemical and/or optical and/or electrical and/or electronic and/or digital and/or data indicators, which alone or in combination may result in the invalidation and/or inactivation of the cartridge.

The cartridges of the present invention may be interrogated by the administration devices according to the present invention prior to injection on one or more of the following levels:
(a) readout of the identity and information storage of the cartridge (*e*.*g*., codes, barcodes, 2D barcodes, 2D data matrix codes, RFID tags, NFC technology and/or data loggers);
(b) detection of a change in one or more indicators resulting from a breach of cartridge integrity and/or prior use in another administration device and/or prior use in the same administration device beyond a predefined time window and/or unauthorised use;
(c) detection of a colour change and/or turbidity change and/or another indicator change in one or more viewing windows of the cartridge, indicating release of one or more marker substances into the drug formulation compartment of the cartridge;
(d) detection of a change in one or more (preferably irreversible) indicator labels and/or (preferably irreversible) thermochromic indicators;
(e) detection of a disturbance of the mechanical and/or physical and/or electrical and/or electronic interaction between the cartridge and the administration device.

This interrogation can be performed online (if the device is connected to the internet) or offline (if the appropriate instructions have been previously downloaded or transmitted to the device).

Certainly, the cartridge itself may have such an interrogation function.

It is preferred that the cartridge of the present invention features at least one unique identifier such as, *e*.*g*., a code (consisting of any element from the group of numbers, letters and/or symbols), a barcode, a 2D barcodes, a 2D data matrix code, an RFID tag and/or NFC technology, which enable tracking and tracing of the cartridge from the moment of release by the manufacturer of the cartridge until the moment of quality control prior to administration.

Barcodes and data matrix codes are state of the art and widely used. RFID- and NFC-based tracking and tracing solutions for pharmaceuticals, also of single units, are known in the art and available, *e*.*g*., from Hanmi Pharmaceutical. The cartridge of the present invention may therefore be trackable and traceable from the time of release from the manufacturer of the cartridge until the time of administration. The verification of the cartridge's identity and track records by interaction with appropriate databases may be performed by different devices along the supply chain (*e*.*g*., RFID storage facilities, such as RFID refridgerators for cold chain products) and finally by use of the cartridge in an administration device according to the present invention.

It is preferred according to the present invention that a breach of cartridge integrity and/or prior use in another administration device and/or prior use in the same administration device beyond a predefined time window and/or unauthorised use and/or the end of designated use may be electrically or electronically sensed and transmitted to one or more data storage devices of the cartridge, *e*.*g*., an RFID tag, which store the identification and integrity data of the cartridge. Electronic recording of such a breach of integrity (or end of designated use) will lead to invalidation of the cartridge and rejection by any suitable administration device. In addition, such information may be transmitted to appropriate databases, thereby electronically invalidating the cartridge also in the databases.

It is further preferred that a breach of cartridge integrity and/or prior use in another administration device and/or prior use in the same administration device beyond a predefined time window and/or unauthorised use may lead to a physical disruption and/or opening of a compartment within the cartridge, from which one or more non-toxic marker dyes or turbidity-inducing substances is or are released into the compartment containing the drug formulation. Preferably, such dyes or other marker substances have already been used in diagnostic and/or therapeutic interventions in human patients and have been demonstrated to be safe when applied via the route of administration intended by use of the cartridge. Examples for such dyes, which could be suitable for invalidating injectables, include, but are not limited to, indocyanine green, indigo carmine, methylene blue, fluorescein or dyes used in the nutrition industry. A change of colour and/or fluorescence and/or turbidity of the drug formulation by release of the marker substance(s) may preferably have one or both of the following consequences: (1) the changed colour and/or turbidity becomes visible in one or more viewing windows of the cartridge and warns the patient not to use the cartridge, (2) the administration device detects the change in colour and/or fluorescence and/or turbidity in the viewing window(s) and rejects the cartridge. In the unlikely event that these controls and/or their detection should fail and the drug formulation were accidentally administered anyway, the marker substances should pose no additional health risk to the patient.

Thus, it is preferred that one or more of the indicator technologies described herein indicate a breach of cartridge integrity and/or prior use of a cartridge in another administration device and/or in the same administration device beyond a predefined time period and/or unauthorised use, leading to the invalidation and/or inactivation of the cartridge, and -just as any administration event - any invalidation event may be reported to the appropriate databases.

In addition to cases in which the cartridge is irreversibly invalidated, the use of the cartridge may also be reversibly and/or temporarily blocked due to administration criteria defined by the administration programme, which have to be met to enable administration. For example, the optimal administration temperature of a drug formulation contained in the cartridge may be significantly higher (*e*.*g*., room temperature) than its storage temperature (*e*.*g*., 2-8°C for cold chain products). If the temperature of the cartridge and/or the drug formulation is too low for administration (*e*.*g*., because it has just been removed from a refridgerator), the cartridge may not be usable until the correct administration temperature has been reached (*e*.*g*., by incubation at room temperature until the cartridge and the drug formulation contained in the cartridge have reached room temperature).

It has to be noted that if this text mentions an invalidation of the cartridge of the invention, the person skilled in the art knows that the same effect can be obtained if a (previously and/or by default) inactivated cartridge is not activated in the mentioned situation. If an irreversible inactivation is mentioned, the same effect can be obtained by irreversibly blocking the activation of a cartridge which is inactive by default unless activated.

It is alternatively or additionally preferred that a breach of cartridge integrity and/or prior use in another administration device and/or prior use in the same administration device beyond a predefined time window and/or unauthorised use may lead to a disturbance of the mechanical and/or physical and/or electrical and/or electronic interaction between the cartridge and the administration device. For example, use of a cartridge of the present invention in an unauthorised administration device may irreversibly damage a delicate part of the cartridge, *e*.*g*., by physical damage to the mechanical interaction interface of the cartridge with the administration device. This may lead, *e*.*g*., to a loss of dosing functionality and/or precision of the cartridge, even if all electronic safety layers are overridden by an unauthorised injection device, *e*.*g*., by a counterfeit injection device.

The cartridge may also be constructed in such a fashion that it is permanently (*e*.*g*., mechanically and/or electronically) locked and only unlocked by interaction with a suitable administration device. Such a locked state may be, *e*.*g*., characterised by a mechanical lock of the cartridge piston or by a lock of the needle unit, in the case that the needle unit is permanently fixed to the cartridge.

The invalidation and/or inactivation mechanisms of the cartridge as described herein can be constructed to exert their function immediately (*e*.*g*., to indicate a physical breach of integrity of the compartment containing the drug formulation or in the moment of ejection of the cartridge from the administration device) and/or after a predefined time period (*e*.*g*., to ensure that the cartridge is not re-used after the execution of an administration programme).

In order to indicate potential breaches of the temperature thresholds defined for the particular cartridge and/or contents of the cartridge (*e*.*g*., breach of a cold chain temperature corridor by freezing or heating), the cartridges of the present invention preferably feature (preferably irreversible) temperature indicator labels and/or (preferably irreversible) thermochromic indicators. Such labels and/or indicators may be applied to or incorporated into the cartridges by methods known in the art (see, *e*.*g*., a detailed description for the analogous application of thermochromic indicators on autoinjectors in PCT/GB2008/001871, p. 6 line 21 to p. 10 line 19, as well as in the literature cited therein). Suitable (preferably irreversible) thermochromic labels and/or other indicator labels and/or other indicators are currently marketed, *e*.*g*., by Cole-Palmer, DeltaTrak, Siltech, LCR Hallcrest, American Thermal Instruments and Skyrad.

Miniature temperature loggers using RFID, NFC or other technologies are suitable for preferred embodiments of the present invention and are currently available both off the shelf and in customised form, *e*.*g*., from the following companies: AMS (*e*.*g*., SL13A or SL900A), Delta Microelectronics (*e*.*g*., D14TEMP01), CAEN RFID (*e*.*g*., easy2log^{®} A927Z), Phase IV Engineering (*e*.*g*., Micro-T™ system, customised to the desired temperature range), Microsensys (*e*.*g*., TELID^{®}311), Maxim Integrated (*e*.*g*., the iButton^{®} series) or PSI Technics (*e*.*g*., the PSI Data Logger systems). Such temperature loggers may be fully passive (*e*.*g*., harvesting energy from reader fields), semi-passive or active. Batteries and/or antennas, if applicable, may be included in the encasing of the temperature logger (*e*.*g*., in the easy2log^{®} A927Z system by CAEN RFID), or the system may be cartridge-specific and adapted to the shape of the cartridge using several separate components, *e*.*g*., a sensor chip (*e*.*g*., SL13A or SL900A from AMS or D14TEMP01 from Delta Microelectronics), a battery, an antenna and/or other elements necessary for the function of the temperature logger. The memory of the temperature logger may be constructed in a tamper-proof fashion by suitable hardware and software engineering known in the art. Logging other parameters such as, *e*.*g*., humidity or physical perturbations, by similar devices is also within the scope of the present invention.

Along the life cycle of a cartridge, the information stored in the memory of its electronic components and/or the readout of its indicators can be controlled and/or monitored and/or interrogated by machines and/or humans.

The cartridges of the present invention may comprise compartments for drug formulations made of any suitable material. In addition to suitable glass compositions, polymeric materials, such as, *e*.*g*., cyclic olefin polymers (*e*.*g*., Daikyo Crystal Zenith®; DeGrazio and Paskiet 2012: The glass quandary; Contract Pharma, 01/02, 2012) are also within the scope of the present invention.

The cartridges of the present invention are preferably partially or fully recyclable in analogy to the recycling process of, *e*.*g*., printer cartridges. In a preferred embodiment, the compartment containing the drug formulation can be separated from the other hardware of the cartridge.

Preferably, the cartridges of the invention are equipped with one or more additional components required for the functionality of the administration device, *e*.*g*., a needle or any other hypodermic injection device. In another preferred embodiment, the administration device necessary for using the cartridges of the present invention may feature built-in components for the administration of the drug formulation contained in the cartridges, or such administration components may be additionally inserted into the administration device. For example, for use according to the present invention, an injection device may be loaded with a single-use hypodermic injection needle and a cartridge of the present invention.

The present invention also pertains to the use and interaction of the cartridges of the present invention with suitable administration devices. Such administration devices may have one or more of the following features and functionalities:
(a) sensors for reading out and/or detecting the identity and integrity information stored in and/or on the cartridge and/or any indicator element of the cartridge, such as, *e*.*g*., optical sensors to verify the identity of the cartridge and/or to read out an optical tracking history of the cartridge, optical sensors to detect colour changes of (preferably irreversible) indicator labels (*e*.*g*., temperature indicator labels) and/or (preferaby irreversible) thermochromic indicators, optical sensors to detect changes in colour and/or turbidity of the drug formulation contained in the cartridge via one or more viewing windows, electric and/or electronic sensors to detect changes in the information stored in any information storage device of the cartridge (*e*.*g*., an RFID tag), mechanical sensors to detect physical and/or mechanical changes of the cartridge (*e*.*g*., detection of previous use or physical damage to the cartridge);
(b) one or more features of contemporary smartphones, such as, *e*.*g*., mobile and internet communication interfaces, applications for decoding of barcodes and/or 2D data matrix codes, a camera and applications for processing images taken by the camera, geotagging functionality and the like;
(c) wireless or wired connectivity, such as, *e*.*g*., WLAN and/or Bluetooth and/or WIFI and/or LAN and/or USB connectivity, for short-range or medium-range interaction with other devices, *e*.*g*., smartphones, tablets, notebooks, personal computers or other personal assistance devices, measurement devices for determining one or more parameters and/or biomarkers feeding into the decision on the dose to be administered from the cartridge and/or any other devices used by healthcare specialists;
(d) internet connectivity, *e*.*g*., via WLAN, WIFI, LTE or any other mobile communication network.

It is preferred that use of the connectivity of an administration device according to the present invention enables tracking and tracing of the cartridge from the time of release from the manufacturer of the cartridge until the time of administration as well as any other dosing, interactive or quality-control features described herein. In this context, the interaction of the cartridge as part of the "internet of things" with any sensor/detector system of transportation and/or storage devices along the supply chain (*e*.*g*., RFID storage facilities, such as RFID refridgerators for cold chain products) is also within the scope of the present invention.

It is preferred that the use and interaction of the cartridges of the present invention with suitable administration devices is controlled by a unique patient identifier. The patient identifier may be a code which has to be entered by the patient via the interaction surface of the administration device, *e*.*g*., a personal identification number as used to unlock smartphones. In a preferred embodiment, the patient identifier is a largely tamper-proof biometric identifier such as, *e*.*g*., a fingerprint scan performed by the administration device. Two or more biometric or non-biometric identifiers can be combined to enhance security and/or to provide an emergency access in case one identifier fails (*e*.*g*., registration of several fingerprints for the event of finger injury, securely stored mastercodes in the event of the patient forgetting the personal identification code, and the like).

It is preferred that the administration of a personalized dose of the drug formulation contained in a cartridge to a particular patient is based on a disease symptom and/or a laboratory parameter, *e*.*g*., a drug level and/or any other biomarker from the patient's blood, urine, stool, breath or tissue that have been previously measured by any device (including, but not limited to, point of care devices and/or devices used by the patient).

The present invention also preferably pertains to the use and interaction of the cartridges of the present invention with suitable administration devices enabling administration of a personalized dose of the drug formulation contained in the cartridge to a particular patient defined and/or qualified and/or cleared and/or directly controlled and/or remote-controlled by the attending physician and/or other healthcare specialists and/or computer programmes and/or computer algorithms. The dosing instructions for the administration device may be defined by and/or transmitted from, *e*.*g*., the office of the attending physician and/or the office of other healthcare specialists and/or a computer programme and/or a computer algorithm and/or a database via the internet, mobile communication or other means of data transfer. Data transfer may be performed using separate channels in parallel or alternatively in case one channel is not available (*e*.*g*., transmission via a mobile phone network if no functional internet connection is available). Thus, the device may be remote-controlled to administer a specific dose to a specific patient.

Data transfer may be open or, preferably, encoded. The use of an encoding procedure in which the unique administration device identification is related to patient identity in the receiving database allowing the administration device to send only encoded, depersonalized information is also within the scope of the present invention.

The present invention also preferably pertains to the use and interaction of the cartridges of the present invention with suitable administration devices enabling variable, personalized dosing based on information transmitted by the attending physician and/or other healthcare specialists and/or a computer programme and/or a computer algorithm and/or a database to the administration device and on the prerequisite that the cartridge contains sufficient drug formulation for the execution of a personalized administration programme devised for the patient, the drug and the medical condition to be treated, *e*.*g*., any dose up to the maximum dose defined by the appropriate guidelines and/or healthcare authorities and/or the attending physician and/or other healthcare specialists. This is important to enable the cartridge to administer any dose within the scope of suitable doses.

The present invention also preferably pertains to the use and interaction of the cartridges of the present invention with suitable administration devices enabling a single administration or two or more immediately consecutive administrations per cartridge and administration event (*e*.*g*., several consecutive injections in order to administer a volume which is too large for one injection site) in a predefined time window and/or defined by the administration programme for optimal dosing.

Importantly, the present invention also preferably pertains to the use and interaction of the cartridges of the present invention with suitable administration devices enabling irreversible invalidation and/or inactivation of the cartridge if the cartridge identity and/or integrity has been compromised and/or if the cartridge's temperature has left a predefined window and/or in case of prior use of the cartridge in another administration device and/or in the same administration device beyond a predefined time window and/or in case of unauthorised use, thereby preventing any potentially unsafe administration.

The present invention also pertains to the use and interaction of the cartridges of the present invention with suitable administration devices enabling transmission of information via the administration device directly or indirectly back to the attending physician and/or other healthcare specialists and/or a hospital and/or a medical office and/or the manufacturer of the cartridge and/or the manufacturer of the injection device and/or healthcare authorities and/or third party payers and/or medical or scientific or insurance databases in order to document the administration event, including protection of the patient's identity and data by suitable encoding and anonymisation technologies as described above. In analogy to the data download to the administration device, data upload from the device to a recipient as described herein may also be performed using one or more channels for information transfer in parallel or alternatively in case of a malfunction. By using such technologies, drug administrations with cartridges of the present invention can be billed and paid per administered dose, per cartridge, per administration event, per time interval and the like, enabling tailored, accurate and reliable payment models (in particular, tamper-proof flat pricing models) also for highly expensive drugs.

The information transmitted in such events may include, but is not limited to, the identification of the device and/or the patient and/or the cartridge, the time of administration, the place of administration (*e*.*g*., geotagging data), the actually administered dose (*e*.*g*., the injection volume) and potential deviations from the administration programme. One purpose for the transmission of such information is to enter the information into electronic health care records and/or medical or scientific or insurance databases. In addition, the patient may also be enabled to enter or edit personal information and/or to enter disease- and/or treatment-relevant information for the attention of the attending physician and/or other healthcare specialists. For example, entering such information may assist in scheduling care processes like appointments, diagnostic procedures and/or therapies. Moreover, the administration device may also be used as a unique personal identifier or a key to unlock information in a healthcare provider's database or in depersonalized detailed health care data. Finally, the administration device may also be equipped with an alarm function to establish an emergency contact between the patient and healthcare practitioners and/or to transmit emergency information to healthcare practitioners and/or rescue services.

Taken together, the administration devices described herein used with the cartridges of the present invention may function as information hubs and bridging devices, which allow physicians or other healthcare specialists to interact with the patient and the patient's data in a safe fashion and to enable and monitor personalized dosing of drug formulations.

### FIGURES

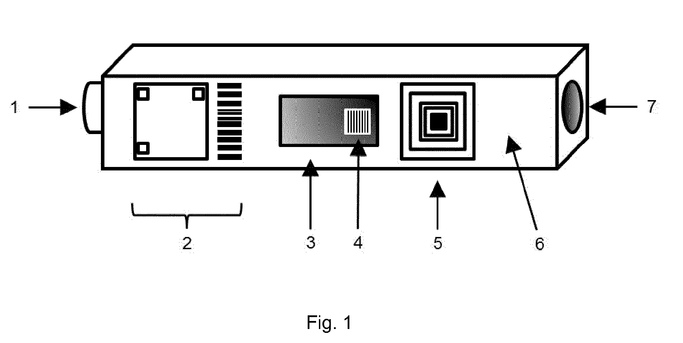

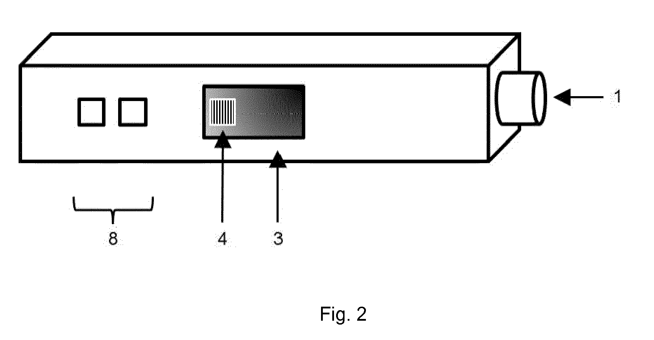

Figures 1 and 2 show opposite sides of an exemplary and schematic representation of a cartridge according to the present invention. The side depicted in Figure 1 features the identify interrogation interfaces and an optional RFID tag and/or temperature logging unit. The opposite side depicted in Figure 2 features the integrity and temperature interrogation interfaces. In the figures, the numerals have the following meaning:
1 = sealed outlet fitting into needle unit;
2 = 2D data matrix code and barcode ID;
3 = viewing window, a part of which stays visible for the user when the cartridge is inserted into or part of an administration device;
4 = measurement area for colour and/or turbidity of the drug formulation in that part of the viewing window which is not visible to the user when the cartridge is inserted into or part of an administration device, as the device's emission and/or detection units needed to measure colour and/or turbidity will block the view;
5 = optional RFID tag or RFID temperature sensor tag or other temperature logger;
6 = inside this part of the cartridge the following components may be located: dye compartment, battery and/or mechanical interface for plunger;
7 = sealed opening for pen plunger fitting into cartridge piston;
8 = irreversible thermochromic indicators and/or temperature labels, *e*.*g*., one indicating exposure to temperatures below the specifications of the cartridge and another for indicating exposure to temperatures above the specifications of the cartridge.

When a cartridge of the present invention is inserted into or is part of an administration device according to the invention, the indicated parts of the cartridge may interact with the device or parts of the device as described in Example 1. However, no part of the exemplary Figures 1 or 2 shall be construed to be limiting to different embodiments of the present invention.

### EXEMPLIFICATION

There are variable possibilities to advantageously develop, and develop further, the teachings of the present invention. For this purpose, reference is made to the examples below, which describe the invention in a representative way. It will be clear that the invention can be practiced otherwise than as particularly described in the foregoing description and in the following examples. Numerous modifications and variations of the present invention are possible in light of the above teachings and, therefore, are within the scope of the appended claims.

### Example 1:

In one embodiment of the present invention, a medication cartridge is constructed for the injection of a liquid biologic drug formulation requiring cold chain storage (2-8°C). The cartridge comprises a safety-sealed glass or polymeric compartment containing a volume of the drug formulation which exceeds the maximum recommended single dose for the disease to be treated. In addition, the cartridge comprises the following identification and surveillance features:
(a) barcode and 2D data matrix code as two redundant unique identifiers of the cartridge;
(b) two irreversible thermochromic indicators or temperature labels for indicating if the temperature of the cartridge has dropped below 2°C or risen above 8°C at any time;
(c) a compartment containing a marker dye;
(d) two viewing windows on opposite sides of the cartridge, enabling visual inspection by the user (patient) and light transmission analysis by the injection device.

This cartridge is prescribed by a physician and obtained by a patient in a pharmacy, transported home in a cool bag and intermediately stored in the patient's household refridgerator at a temperature of 4-7°C.

The attending physician notifies the patient that, according to the laboratory results of a blood sample drawn at the last visit of the patient in the outpatient clinic, drug levels of the biologic were lower than expected and levels of a disease activity biomarker have increased. In addition, the attending physician asks the patient to report current symptoms and personal observations. As it turns out, the laboratory results are in good agreement with a slight worsening of disease symptoms experienced by the patient and reported to the physician. Therefore, the attending physician provides a new injection programme (coding for administration of a dose which is slightly higher than the last one) on a secure website of the hospital outpatient clinic for download to the patient's self-injection device. The injection protocol requires two subsequent subcutaneous injections of 0.68 ml of the liquid drug formulation, corresponding to 45.3% of the authorised maximum dose deliverable with this particular single-use cartridge (3 ml).

The patient unlocks his personal self-injection device by a fingerprint scan and downloads the injection protocol from the hospital's website to the device. The device notifies the patient that its self-test was uneventful, that its battery status is sufficient and that the device is ready for executing the downloaded injection programme. The interaction of the device with the patient is optionally visual and/or by voice guidance.

The patient visually inspects the cartridge according to the guidance provided by the self-injection device and/or by other information provided by the manufacturer of the cartridge. If the patient finds no indication of a breach of cartridge integrity, in particular no colouring of the irreversible thermochromic indicator fields or of the liquid drug formulation, the patient inserts the cartridge into the self-injection device. The device reminds the patient to attach a suitable injection needle unit to the device. After another self-test to ensure proper attachment of both the needle unit and the cartridge, the self-injection device performs the following verification and quality control steps:
(a) scanning of the barcode and 2D data matrix code and comparison of the data with an internal (downloaded) and/or external database;
(b) optical analysis of the irreversible thermochromic indicator fields on the cartridge;
(c) optical analysis of the colouring of the liquid drug formulation by light transmission and analysis using the two viewing windows of the cartridge.

After the cartridge has passed all identity and quality tests, the device instructs the patient to perform the two injections within a time window of one minute per injection site.

Optionally, all guidance and interaction steps apart from the injection can also be performed and/or visualised on a personal digital assistance device of the patient, e.g., a smartphone or a tablet computer, which is connected to the self-injection device by Bluetooth or WLAN.

After performing the injection, the patient removes the needle unit and the cartridge from the self-injection device. Removal of the cartridge from the device ruptures the marker dye compartment and irreversibly invalidates the cartridge and the remaining drug formulation. Even if the cartridge were opened and the drug formulation were extracted for unauthorised use, the strong colouring of the solution would immediately indicate tampering and/or quality issues even to an untrained observer. Finally, the patient safely discards the shielded needle unit in an appropriate waste container and collects the cartridge for recycling (which is performed by sending cartridges in postage-paid padded envelopes back to the manufacturer). If necessary, the patient performs cleaning or maintenance tasks to keep the device in optimal condition for the next injection.

### Example 2:

In another embodiment of the present invention, the cartridge of Example 1 is equipped with a passive RFID tag (instead of a barcode) together with a 2D data matrix code, enabling more detailed data storage and, *e*.*g*., addition of tracking history to the memory of the cartridge. In addition, information regarding the use of the cartridge in a self-injection device will be irreversibly stored on the RFID tag to prevent accidental or unauthorised (re)use of the cartridge. In an alternative embodiment, the self-injection device irreversibly destroys the RFID tag after injection.

### Example 3:

In a further embodiment of the present invention, the cartridges described in Example 1 or 2 are equipped with a semi-passive or active RFID sensor tag for temperature logging or with another temperature logging device. In yet another embodiment of the present invention, the electronic temperature logging system is not installed in addition to but instead of the irreversible thermochromic indicators or temperature labels as described in Example 1.

### Example 4:

In a further embodiment of the present invention, the cartridges described in any one of Examples 1 to 3 are equipped with a time-dependent dye release functionality for invalidation of the cartridge in case of significant exceedance of the injection time limit (*e*.*g*., if a user accidentally leaves the cartridge in the self-injection device after injection instead of removing it). In addition to the electronic blockade of further use of the cartridge in the same self-injection device by a database entry recording the injection and/or invalidation of the data storage unit(s) of the cartridge (*e*.*g*., the RFID tag), an additional safety layer in case of malfunction of the electronic blockade consists of a mechanism which leads to a controlled disruption of the marker dye compartment in such a fashion that the marker dye is only released after a defined time limit, *e*.*g*., by a slow chemical reaction and/or a physical or mechanical installation.

## Claims

1. A medication cartridge which
(a) contains sufficient drug formulation for the execution of an administration programme devised for the drug and the medical condition to be treated;
(b) comprises means to enable programmed administration of a personalized dose of the drug formulation to a patient, comprising the use of the cartridge in one or more administration events;
(c) comprises means to invalidate and/or inactivate the cartridge (i) after use according to (b) and/or (ii) by any breach of cartridge integrity and/or (iii) by unauthorised use, or comprises means to activate the cartridge if none of (i) - (iii) is detected;
(d) is adapted for use together with a suitable administration device or comprises an administration unit.

2. The cartridge according to claim 1 comprising at least one unique identifier such as a code consisting of any element from the group of numbers, letters and/or symbols, a barcode, a 2D barcode, a 2D data matrix code, an RFID tag and/or NFC technology, which enable tracking and tracing of the cartridge.

3. The cartridge according to claim 1 or 2 comprising one or more indicators selected from the group consisting of mechanical, physical, chemical, optical, electrical, electronic, digital and data indicators for prior use and/or unauthorised use of the cartridge according to claim 1 and/or any breach of cartridge integrity.

4. The cartridge according to claim 3, in which the indicators are irreversible.

5. The cartridge according to any one of claims 1 to 4 comprising a temperature indicator label and/or thermochromic indicator and/or a temperature-logging unit coupled with means for invalidation and/or inactivation of the cartridge in case of a breach of a defined temperature threshold.

6. The cartridge according to any one of claims 1 to 5 for administration of a personalized dose of the drug formulation to a particular patient, wherein the dose is dependent of one or more unique patient identifiers.

7. The cartridge according to any one of claims 1 to 6 for administration of a personalized dose of the drug formulation to a particular patient, wherein the dose is dependent of a disease symptom and/or a laboratory parameter and/or a biomarker.

8. The cartridge according to any one of claims 1 to 7 for administration of a personalized dose of the drug formulation to a particular patient, wherein the dose is dependent of being defined and/or qualified and/or cleared and/or directly controlled and/or remote-controlled by the attending physician and/or another healthcare specialist and/or a computer programme and/or a computer algorithm.

9. The cartridge according to claim 8, wherein the cartridge contains sufficient drug formulation for the execution of a personalized administration programme devised for the patient, the drug and the medical condition to be treated.

10. The cartridge according to any one of claims 1 to 9 for a single administration or two or more preferably immediately consecutive administrations and wherein each administration event is in a predefined time window and/or defined by the administration programme for optimal dosing.

11. The cartridge according to any one of claims 1 to 10 comprising means for tracking and tracing of the cartridge from the time of release from the manufacturer of the cartridge until the time of administration.

12. The cartridge according to any one of claims 1 to 11 comprising means for invalidation and/or inactivation of the cartridge if the cartridge integrity has been compromised and/or if the cartridge's temperature has left a predefined window and/or in case of prior use of the cartridge in another administration device and/or in case of prior use in the same administration device beyond a predefined time window and/or in case of unauthorised use.

13. The cartridge according to any one of claims 1 to 12 adapted for transmission of information via a transmission unit directly or indirectly back to the attending physician and/or other healthcare specialists and/or a hospital and/or a medical office and/or the manufacturer of the cartridge and/or the manufacturer of the administration device and/or healthcare authorities and/or third party payers and/or medical or scientific or insurance databases in order to document the administration event.

14. The cartridge according to any one of claims 1 to 13 comprising means for protection of the patient's identity and data preferably by suitable coding and anonymisation technologies.
